# EUROPEAN PATENT SPECIFICATION

(11) **EP 4 511 796 B1**
(45) Date of publication and mention of the grant of the patent: **11.02.2026**
(21) Application number: 23722691.5
(22) Date of filing: 17.04.2023
(51) Int. Cl.: G06T 7/00, G06T 7/60, G06T 7/62

(54) **"METHOD TO ESTIMATE IN REAL TIME THE LIKELIHOOD OF SUCCESS OF THROMBECTOMY SURGERY"**
VERFAHREN ZUR ECHTZEITSCHÄTZUNG DER WAHRSCHEINLICHKEIT DES ERFOLGS EINER THROMBEKTOMIEOPERATION
MÉTHODE D'ESTIMATION EN TEMPS RÉEL DE LA PROBABILITÉ DE RÉUSSITE D'UNE CHIRURGIE DE THROMBECTOMIE

(30) Priority: 20.04.2022 IT 202200007838
(43) Date of publication of application: 26.02.2025
(73) Proprietor: Politecnico di Milano, 20133 Milano (IT)
(72) Inventor: MIGLIAVACCA, Francesco, 20133 Milano (IT); RODRIGUEZ MATAS, Jose Felix, 20133 Milano (IT); BRIDIO, Sara, 20133 Milano (IT); LURAGHI, Giulia, 20133 Milano (IT)
(74) Representative: Perani & Partners S.p.A.
(86) International application number: PCT/IB2023/053915
(87) International publication number: WO 2023/203463

(56) References cited:
- HOLSWILDER GHISLAINE ET AL: "The prognostic value of extracranial vascular characteristics on procedural duration and revascularization success in endovascularly treated acute ischemic stroke patients", vol. 7, no. 1, 8 February 2022 (2022-02-08), pages 48 - 56, XP055979691, ISSN: 2396-9873, Retrieved from the Internet <URL:http://journals.sagepub.com/doi/full-xml/10.1177/23969873211067662> [retrieved on 20221109], DOI: 10.1177/23969873211067662
- VELASCO GONZALEZ AGLAÉ ET AL: "Predictors of Successful First-Pass Thrombectomy with a Balloon Guide Catheter: Results of a Decision Tree Analysis", TRANSLATIONAL STROKE RESEARCH, SPRINGER US, BOSTON, vol. 11, no. 5, 23 May 2020 (2020-05-23), pages 900 - 909, XP037242727, ISSN: 1868-4483, [retrieved on 20200523], DOI: 10.1007/S12975-020-00784-2
- TEO YAO HAO ET AL: "Predicting Clinical Outcomes in Acute Ischemic Stroke Patients Undergoing Endovascular Thrombectomy with Machine Learning", CLINICAL NEURORADIOLOGY, SPRINGER BERLIN HEIDELBERG, BERLIN/HEIDELBERG, vol. 31, no. 4, 24 January 2021 (2021-01-24), pages 1121 - 1130, XP037636855, ISSN: 1869-1439, [retrieved on 20210124], DOI: 10.1007/S00062-020-00990-3
- LURAGHI GIULIA ET AL: "The first virtual patient-specific thrombectomy procedure", JOURNAL OF BIOMECHANICS, PERGAMON PRESS, NEW YORK, NY, US, vol. 126, 10 July 2021 (2021-07-10), XP086785472, ISSN: 0021-9290, [retrieved on 20210710], DOI: 10.1016/J.JBIOMECH.2021.110622
- JONG-WON CHUNG ET AL: "Characterization of clot composition in acute cerebral infarct using machine learning techniques", ANNALS OF CLINICAL AND TRANSLATIONAL NEUROLOGY, vol. 6, no. 4, 4 March 2019 (2019-03-04), GB, pages 739 - 747, XP055650230, ISSN: 2328-9503, DOI: 10.1002/acn3.751

## Description

### SCOPE OF APPLICATION

The present invention relates to a method to estimate in real time the likelihood of success of thrombectomy surgery.

More in detail, the present invention concerns a method that allows to calculate the likelihood of success of a thrombus removal surgery, for example using a certain procedure performed with a respective biomedical device.

### Description of the prior art

It is known in the art to perform thrombectomy surgery, i.e. a surgical procedure that provides for the removal of a thrombus occluding an arterial or venous vessel, and finds for example application for large arterial vessels of the limbs, coronary, carotid arteries, intracranial vessels, arteries of the lower limbs.

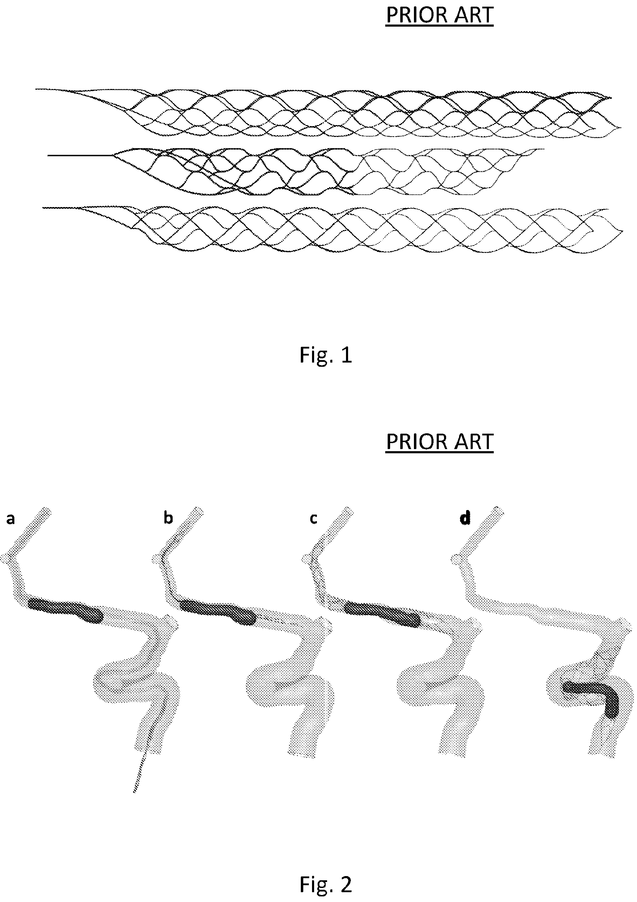

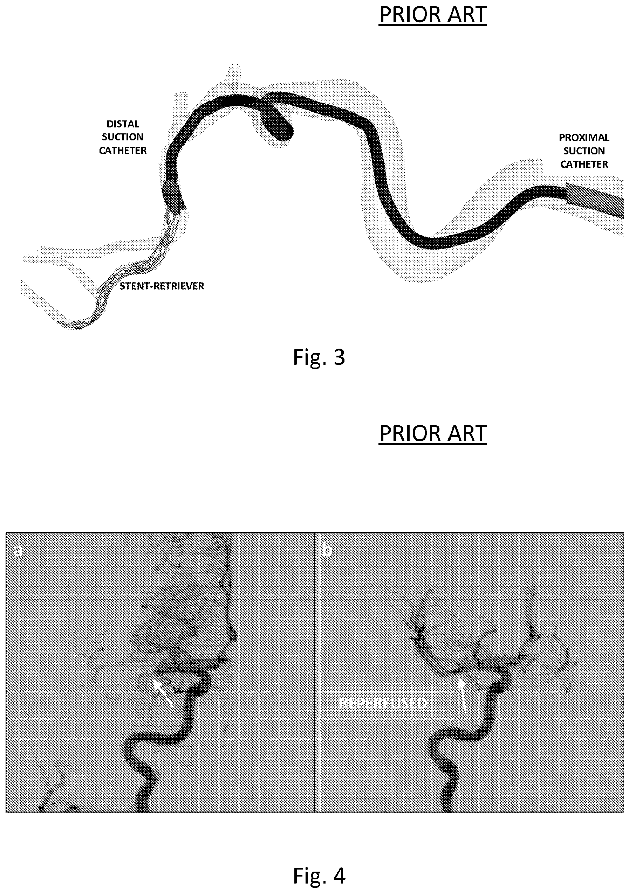

Mechanical thrombectomy is a treatment for acute ischemic stroke, a pathology that occurs when a thrombus occludes a cerebral artery and prevents blood perfusion of the cerebral tissues downstream of the occlusion (Figure 4a). Thrombectomy aims to mechanically remove the thrombus, through a minimally invasive procedure. The procedure may use a biomedical device such as a stent-retriever (Figure 1), i.e. a metallic mesh generally made of nickel-titanium alloy that traps the thrombus, or suction catheters, or a combination of the two devices. The procedure provides for accessing the patient's vascular network usually from the femoral artery with a catheter, which is positioned as far as the occlusion site. **In** the case of thrombectomy with stent-retriever, the stent is constrained inside the catheter (Figure 2a) and positioned at the occlusion (Figure 2b). The catheter is then retrieved, freeing the stent, which tends to recover its expanded shape, thus capturing the thrombus (Figure 2c). The thrombus-stent complex is finally retracted (Figure 2d), freeing the artery (Figure 4b).

In the case of thrombectomy with suction catheter, a distal catheter is advanced until coming into contact with the thrombus. Suction is activated manually with syringes or through suction pumps and the thrombus is totally or partially sucked in. The catheter is then retracted until it reaches a second, larger proximal catheter placed at the base of the carotid artery. Combined techniques are known that can provide for the use of stent-retriever and proximal catheter or stent-retriever, distal catheter and proximal catheter (Figure 3).

A method to estimate in real time the likelihood of success of thrombectomy surgery is described in HOLSWILDER GHISLAINE ET AL.: "The prognostic value of extracranial vascular characteristics on procedural duration and revascularization success in endovascularly treated acute ischemic stroke patients", EUROPEAN STROKE JOURNAL, vol. 7, no. 1, 8 February 2022. The predictive model proposed in the aforementioned document is based on the analysis of some geometric parameters of the cerebral vessels and trained on retrospective clinical data of patients with acute ischemic stroke subjected to thrombectomy procedure.

### Problem of the prior art

In the prior art, finite element analyses are used in the development of the biomedical devices and of their procedures that are to be used in thrombectomy surgeries. In fact, finite element computational modellings are used to simulate the behaviour of devices and procedures in order to evaluate surgery situations. However, these are very expensive computational systems that require high computational costs, and therefore can be used in the step of research, development and optimization of devices and their procedures, but not to evaluate surgery strategies immediately before surgery is performed. In practice, the patient should be operated on shortly after arrival at the hospital and the identification of the thrombus.

### SUMMARY OF THE INVENTION

Aim of the invention in question is to be able to have a method that allows to quickly provide a reliable and trustworthy evaluation of the likelihoods of success of thrombectomy surgery in order to decide the best surgery strategy for the patient to be operated on.

The technical task set and the objects specified are substantially attained by a method comprising the technical features as set out in one or more of the appended claims.

### Advantages of the invention

Thanks to one embodiment, it is possible to obtain a method that allows to provide a real-time estimate of the likelihoods of success of thrombectomy surgery, offering a surgeon the possibility to select before surgery the procedure and the biomedical devices that offer greater chances of success.

### BRIEF DESCRIPTION OF THE DRAWINGS

The characteristics and advantages of the present invention will be apparent from the following detailed description of possible practical embodiments, illustrated by way of non-limiting example in the set of drawings, in which:
- Figure 1 shows some examples of stent-retrievers, i.e. known biomedical devices,
- Figure 2 shows an example of a computational model of the known procedure of thrombectomy with stent-retriever: a) constraint of the stent, b) positioning near the thrombus, c) release of the stent with thrombus capture and d) retraction of the stent with thrombus transported outside the artery,
- Figure 3 shows an example of a computational model of the known procedure of combined thrombectomy with stent-retriever, distal and proximal catheter of known type,
- Figure 4 shows some angiographic images a) before and b) after a thrombectomy surgery according to the prior art,
- Figure 5 shows a flowchart of an application example of the method of the present invention,
- Figure 6 shows an example of application of some steps of the method of the invention,
- Figure 7 shows an example of execution of a step of the method of the invention,
- Figure 8 shows an example of execution of a further step of the method of the invention,
- Figure 9 shows an example of fragmentation of the thrombus during the procedure, according to the prior art,
Figure 10 shows an example a) of non-optimal distal positioning of a stent-retriever and b) of correct positioning of a stent-retriever, according to the prior art.

### DETAILED DESCRIPTION

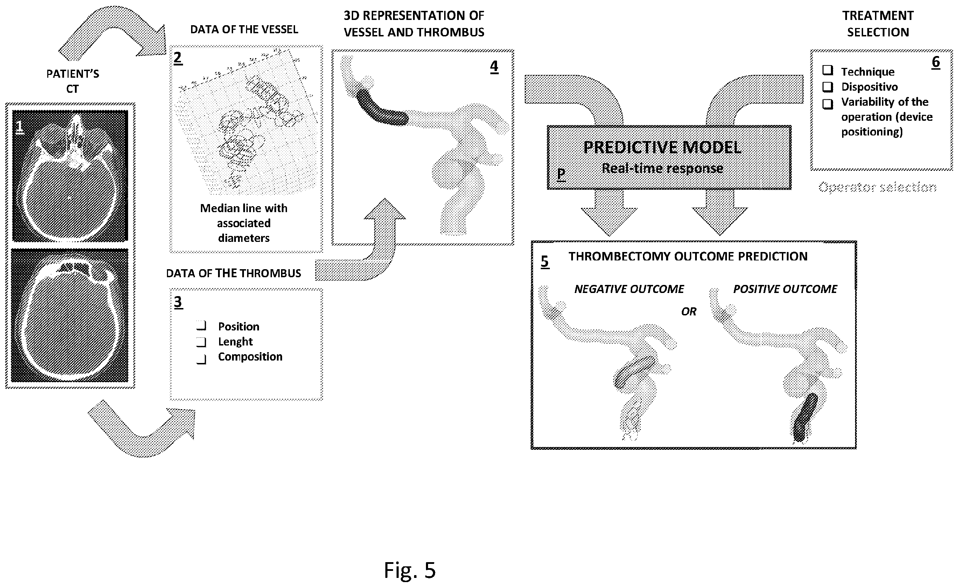

The present invention relates to a method to estimate in real time the likelihood of success of thrombectomy surgery, which allows to provide an estimate of the likelihood of success of the procedure of removing an occlusion, for example, from a blood vessel. Figure 5 shows an example of application of the method of the invention in a thrombectomy surgery for the removal of cerebral thrombi.

The method comprises the step of creating a database generated from thrombectomy patterns and training a predictive algorithm P with that data using a processing unit associated with the database. In other words, the method provides for training the predictive algorithm P according to machine learning techniques on the basis of data precalculated from the thrombectomy patterns, preferably comprising one or more of clinical images, data concerning the geometry of the occluded vessels, the geometry of the thrombus and its composition, the biomedical devices and the procedures used in thrombectomy surgery and the outcome of such surgery.

The step of creating a database generated from the thrombectomy patterns and training a predictive algorithm P using a processing unit associated with the database comprises training the predictive algorithm P by processing the data using machine learning techniques on a high number of finite element numerical simulations of the thrombectomy surgery. In other words, the data are generated from finite element numerical simulations of the thrombectomy surgery.

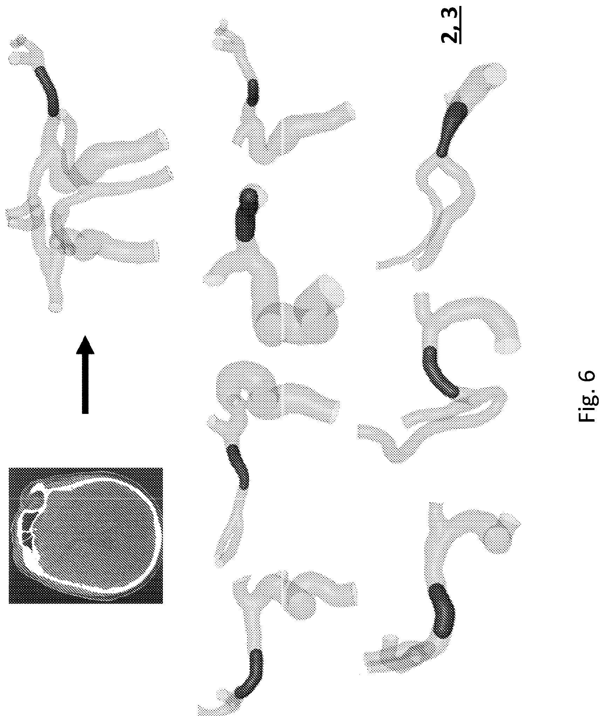

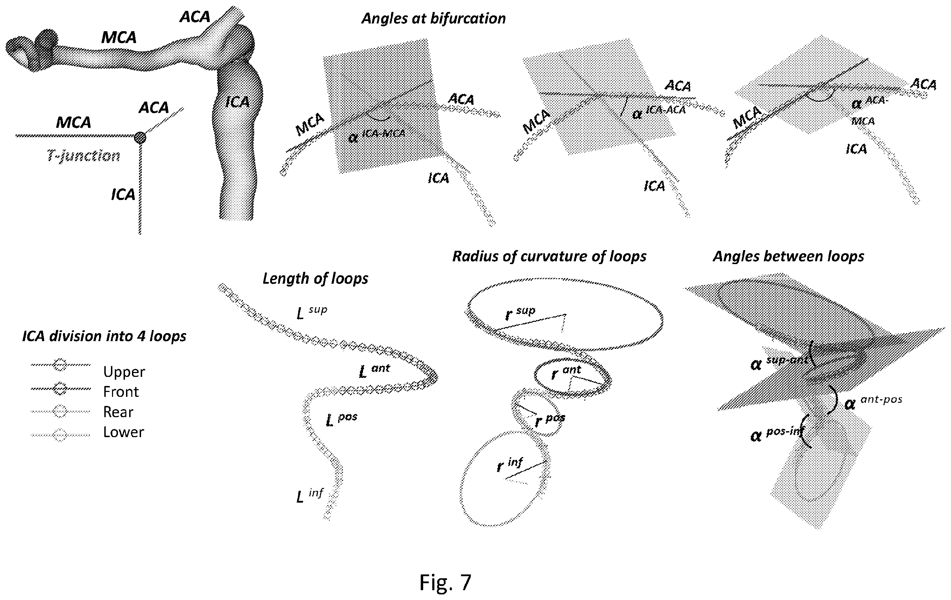

The method comprises the step of acquiring, in real time, clinical images relating to the occluded vessels of a patient. The method provides for extracting from said clinical images both geometric parameters of the occluded vessels 2 and composition parameters of the occlusion 3 (Figure 5). Figure 6 shows an example of execution of this step for extracting from the clinical images of a patient geometric parameters of the occluded vessels (shown in Figure 7) and composition parameters of the occlusion.

The method then comprises the step of generating a three-dimensional model 4 of the occluded vessels and of the occlusion by processing the geometric parameters of the occluded vessels 2 and the composition parameters of the occlusion 3 using the processing unit.

The method also comprises the step of selecting indicator parameters for thrombectomy surgery by processing the three-dimensional model of the occluded vessels and of the occlusion.

The method comprises the step of calculating the likelihood of success 5 of thrombectomy surgery with removal of the occlusion by processing the indicator parameters using the predictive algorithm.

With reference to the example of Figure 5, it is worth noting how the method allows to extract from clinical images by computed tomography (in short CT) of occluded arteries geometric parameters of the vessels and information on the occlusion that, provided in input to a predictive model, allow to provide an estimate of the likelihood of success of the procedure of removing the occlusion from the blood vessel in a thrombectomy surgery for the removal of cerebral thrombi. The method finds application for the removal of occlusions, for example thrombi, of arterial or venous vessels. For example, the method also finds application in the estimate of the likelihood of success of thrombectomy surgery for the removal of occlusions from the coronaries.

In accordance with a first preferred solution of the invention, the step of extracting indicator parameters provides for calculating morphological parameters of the vessels by analysis of the median line of the occluded vessels and their diameters. This step also provides for calculating morphological parameters of the occlusion by analysis of the length, diameter and composition of the occlusion. Figure 7 shows an example of extraction of geometric parameters from the median line of the vessels, wherein the morphological parameters of the vessels preferably comprise one or more parameters chosen from the angles that form where the Internal Carotid Artery ICA bifurcates onto the Anterior Cerebral Artery ACA and Middle Cerebral Artery MCA, the average diameters of the Middle Cerebral Artery MCA and Anterior Cerebral Artery ACA, and characteristic parameters of the carotid siphon of the Internal Carotid Artery ICA. Still preferably, the characteristic parameters of the carotid siphon of the Internal Carotid Artery ICA comprise one or more parameters selected from the radii of curvature of the loops, the length of the loops, the tortuosity of the loops, the angles between the loops and the average diameters of the loops.

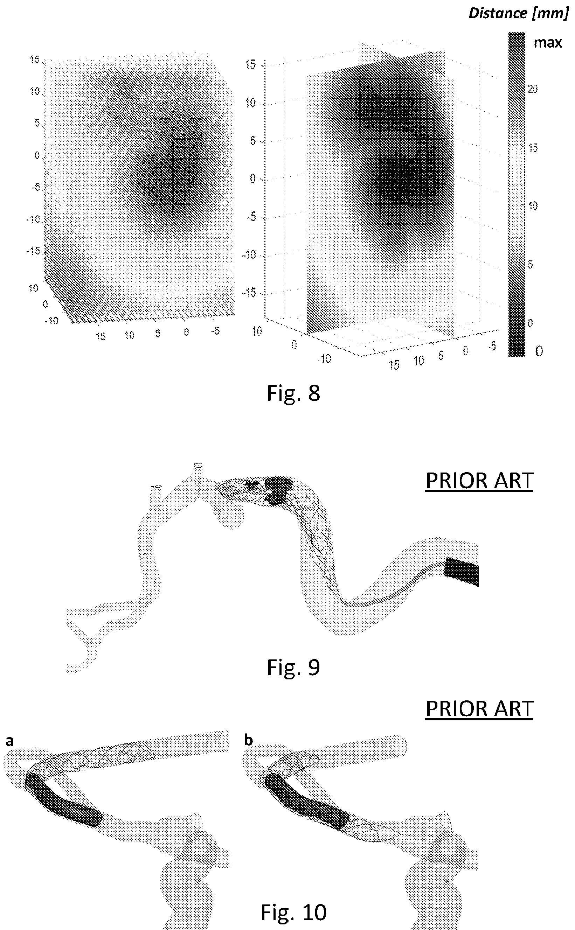

In accordance with a second embodiment alternative to the previous one and illustrated by way of example in Figure 8, the step of extracting indicator parameters provides for calculating characteristic parameters of the geometry of the vessels by analysing the entire geometry of the occluded vessels using the level set technique. This step also provides for calculating morphological parameters of the occlusion by analysis of the length and diameter and composition of the occlusion. This second embodiment is not intended to extract geometric parameters as required by the first embodiment. Instead, it envisages analysing the entire geometry of the vessel. Preferably, again with reference to Figure 8, the step of calculating characteristic parameters of the geometry of the vessels includes defining a fixed volume scaled to accommodate all possible patient vascular geometries. This step also provides for discretising the volume with a grid according to a sensitivity analysis and for placing each reconstructed vascular geometry into the grid in the three-dimensional model of the occluded vessels and of the occlusion and calculating a matrix of the same size as the number of grid points by measuring the distances between each grid point and the nearest point of the vascular geometry. This step also provides for calculating the characteristic parameters of the geometry of the vessels by processing the matrix using the technique of principal component analysis. Summarizing, Figure 8 shows an example of level set application for measuring the distances between the grid points (fixed for all the vascular geometries) and the reconstructed external surface of the vessel. The level set technique consists of defining a fixed volume of appropriate size so as to be able to contain all the vascular geometries of the patients, once aligned with each other. The volume is then discretised, according to a sensitivity analysis, generating the grid. Each reconstructed vascular geometry as explained above, is placed into this grid and the distances between each grid point and the nearest point of the vascular geometry are measured. This yields a matrix of the same large size as the number of grid points, which contains all the information about the examined vascular geometry. This matrix is subjected to a principal component analysis (also called PCA), a method to efficiently reduce the size of a system while maintaining a high percentage of the information contained. This reduces the geometry of the vessel to a low number of parameters. Unlike the first embodiment, the parameters thus obtained do not have a clear and unique physical counterpart, but can represent a set of characteristics. An advantage of this solution is that the parameterization is not done according to parameters established a priori, but by choosing the best reduced representation of the geometry using the PCA. As also happens for the first embodiment solution, the parameters thus extracted, together with the characteristics of the thrombus, are provided in input to the predictive algorithm for calculating the likelihood of success of the thrombectomy surgery.

According to the preferred solution of the invention shown in Figure 5, the clinical images 1 comprise images obtained by computed tomography and/or magnetic resonance imaging.

Still preferably, the geometric parameters of the occluded vessels comprise median line and diameter for each occluded vessel. Still preferably, the composition parameters of the occlusion 3 comprise position, length and composition of the occlusion. In accordance with the preferred solution just described, the step of extracting geometric parameters of occluded vessels 2 and composition parameters of the occlusion 3 from the clinical images provides for processing the clinical images using a grayscale analysis algorithm to extract the composition parameters of the occlusion.

In the example illustrated in Figure 6, the method provides for automatically deriving from clinical images, such as computed tomography or magnetic resonance imaging, the patient-specific geometry of the cerebral artery affected by acute ischemic stroke and thrombus. Median line and diameters are identified for the vessel, from which an automatic procedure allows obtaining the patient-specific three-dimensional reconstruction of the vessel. From the images, position and length of the thrombus are also determined and its composition is estimated based on the grayscale. The 3D representation of the patient-specific vessel and thrombus is obtained by positioning the reconstruction of the thrombus in the vessel.

Preferably, the step of calculating the likelihood of success 5 of thrombectomy surgery with removal of the occlusion by processing the indicator parameters using the predictive algorithm, provides for calculating the likelihood of an occlusal fracture occurring by processing the indicator parameters using the predictive algorithm P (example of Figure 9). Preferably, a thrombus fracture model is also placed into the finite element model of the thrombectomy surgery. By collecting the results of a certain number of simulations of thrombectomy with fracture model it is possible to train a predictive model that, based on indicators such as vascular geometric characteristics, thrombus properties and used thrombectomy technique, provides the likelihood of the thrombus fracture to occur.

With reference to Figure 10, it is worth noting how the method of the invention allows to calculate the likelihood that a thrombectomy surgery, performed with one or more biomedical devices using a certain procedure, can be successfully performed (Figure 10b) with a correct positioning of the stent-retriever with respect to the thrombus. On the other hand, the method also allows estimating the likelihood that the thrombectomy surgery may fail, by optionally specifying what might be the reason for such failure, as shown for example in Figure 10a in which an incorrect positioning of the stent-retriever with respect to the thrombus is illustrated.

In accordance with a preferred solution of the invention, the step of training the predictive algorithm P by processing the data using machine learning techniques on a predefined number of finite element numerical simulations of thrombectomy surgery provides that the thrombectomy surgery uses at least one thrombectomy procedure and at least one respective biomedical device, preferably a plurality of thrombectomy procedures and respective biomedical devices used. Furthermore, before the step of calculating the likelihood of success 5 of thrombectomy surgery, the method provides for the further step of selecting 6 at least one thrombectomy procedure and at least one respective biomedical device to be used in the thrombectomy surgery. This is possible as the predictive algorithm P is trained with databases obtained with finite element computational simulations of different types of thrombectomy procedures, i.e. using different devices, such as different stent-retriever designs and/or suction catheters, and different surgery techniques. For example, the user/surgeon can select a biomedical device, such as a stent-retriever and a respective procedure for thrombectomy surgery from those available in a dedicated list (or library) stored in the database. The library comprises a plurality of biomedical devices and respective surgery procedures for which the predictive algorithm is suitably trained. The method will produce as a result an estimate in real time of the likelihood of success of thrombectomy surgery to be performed on the patient under examination, in the event that the selected device (or the devices if more than one) and the respective thrombectomy procedure are used. The selection of device(s) and procedure may be performed prior to the calculation of the estimate of the likelihood of success of the surgery, so that the calculation is focused on the combination selected by the operator with reduction of computational cost and calculation time. Alternatively, the method can calculate the estimate of likelihood of success of thrombectomy surgery for all combinations of devices and procedures present in the library for which the predictive algorithm has been appropriately trained, so as to automatically identify the device-procedure combination that offers the best likelihood of success for the patient under examination.

It is worth noting that the parameters resulting from the analysis of patient-specific vessel and thrombus are used to query the predictive model on the outcome of a selected thrombectomy surgery. In the example described and illustrated, the predictive model is a surrogate model, i.e. a model built with machine learning techniques on a large number of finite element numerical simulations of the mechanical recanalization procedure of patients affected by acute ischemic strokes, so as to learn to recognize the relationship between a set of input parameters, in this case geometric parameters of the vessel and characteristics of the thrombus, and the output of interest. The model provides in real time the likelihood of success of the surgery procedure, in terms of the likelihood of the thrombus to be removed from the patient's vessel. Optionally, the model may provide the likelihood of the thrombus fracture to occur (Figure 9), which could imply an incomplete recanalization of the vessel. As mentioned before, the predictive model includes a library of possible thrombectomy techniques and device models and sizes. It also provides for the possibility of considering a non-optimal use of the devices, such as a non-optimal positioning of the stent-retriever by the operator (Figure 10a). The model can have two different uses. In the first mode, the operator must indicate the type of procedure that he intends to use in the patient, for example stent-retriever and/or suction catheters, model of the devices used, etc., and the model will provide its likelihood of success. In the second mode, the operator can ask the model to compare the likelihoods of success of all the procedures and their devices contained in the library or of a subgroup thereof, in order to identify the one with the best likelihoods of success in the patient under examination.

Advantageously, the method of the invention is particularly useful in the step of optimization of a device and/or of the procedure but above all as support for the interventional surgeon to choose the optimal device and/or the procedure for the specific patient before surgery.

Advantageously, the method provides for acquiring images and data of the patient from diagnostic tools such as CT and magnetic resonance imaging to build a 3D model of the vessels and of the thrombus, with specific data on its morphological conformation and composition. The artificial intelligence system based on the predictive algorithm, trained on real cases, knows certain devices (e.g., stents) and surgery procedures. As explained above, the surgeon can choose the devices and the procedures to be used in surgery so as to know their likelihood of success, or the system determines the device-procedure combination that has the greatest likelihood of success among those available, that is, for which the artificial intelligence system has been trained.

Advantageously, based on a limited number of anatomical measurements the method is able to faithfully reconstruct the geometry of the patient-specific anatomical district and the position, size and conformation of the thrombus.

Advantageously, the predictive model used in the method of the invention is trained, verified and validated with real clinical data using finite element numerical simulations. In addition, the estimate of the likelihood of success is based on the query of a reduced or surrogate predictive model that provides the result in real time. The method therefore allows to evaluate the performance of a biomedical device, such as a stent-retriever, but also what is the best device to use for the patient considered and the best thrombus extraction technique. The method also makes it possible to estimate the fragmentation of the thrombus during extraction on the basis of the stresses and deformations provided by the simulations.

## Claims

1. Method to estimate in real time the likelihood of success of thrombectomy surgery, comprising the following steps:
- creating a database generated from thrombectomy patterns and training a predictive algorithm (P) with that data using a processing unit associated with the database;
- acquiring clinical images (1) relating to the occluded vessels of a patient and extracting from said clinical images the geometric parameters of the occluded vessels (2) and the composition parameters of the occlusion (3);
- generating a three-dimensional model (4) of the occluded vessels and of the occlusion by processing the geometric parameters of the occluded vessels (2) and the composition parameters of the occlusion (3) using the processing unit;
- selecting indicator parameters for thrombectomy surgery by processing the three-dimensional model of the occluded vessels and of the occlusion;
- calculating the likelihood of success (5) of thrombectomy surgery with removal of the occlusion by processing the indicator parameters using the predictive algorithm (P),
**characterised in that** the step of creating a database generated using thrombectomy patterns and training a predictive algorithm (P) with said data using a processing unit associated with the database, comprises training the predictive algorithm (P) by processing the data using machine learning techniques on a predefined number of finite element numerical simulations of the thrombectomy surgery.

2. Method according to claim 1, wherein the step of selecting indicator parameters comprises the sub-steps of:
- calculating morphological parameters of the vessels by analysis of the median line of the occluded vessels and their diameters;
- calculating morphological parameters of the occlusion by analysis of the length, diameter and composition of the occlusion.

3. Method according to claim 2, wherein the morphological parameters of the vessels comprise one or more parameters chosen from the angles that form where the Internal Carotid Artery (ICA) bifurcates onto the Anterior Cerebral Artery (ACA) and Middle Cerebral Artery (MCA), the average diameters of the Middle Cerebral Artery (MCA) and Anterior Cerebral Artery (ACA), and characteristic parameters of the carotid siphon of the Internal Carotid Artery (ICA).

4. Method according to claim 3, wherein the characteristic parameters of the carotid siphon of the Internal Carotid Artery (ICA) comprise one or more parameters selected from the radii of curvature of the loops, the length of the loops, the tortuosity of the loops, the angles between the loops and the average diameters of the loops.

5. Method according to claim 1, wherein the step of selecting indicator parameters comprises the sub-steps of:
- calculating characteristic parameters of the geometry of the vessels by analysing the entire geometry of the occluded vessels using the level set technique;
- calculating morphological parameters of the occlusion, by analysis of length, diameter and composition of the occlusion.

6. Method according to claim 5, wherein the step of calculating characteristic parameters of the vessel geometry comprises the sub-steps of:
- defining a fixed volume scaled to be able to accommodate all possible patient vascular geometries;
- discretising the volume in a grid according to a sensitivity analysis;
- placing each reconstructed vascular geometry into the grid in the three-dimensional model of the occluded vessels and of the occlusion and calculating a matrix of the same size as the number of grid points by measuring the distances between each grid point and the nearest point of the vascular geometry;
- calculating the characteristic parameters of the geometry of the vessels by processing the matrix using a technique of principal component analysis.

7. Method according to any one of the preceding claims, wherein the clinical images (1) comprise images obtained by computed tomography and/or magnetic resonance imaging.

8. Method according to any one of the preceding claims, wherein
- the geometric parameters of the occluded vessels comprise median line and diameter for each occluded vessel;
- the composition parameters of the occlusion (3) comprise position, length and composition of the occlusion.

9. Method according to claim 8, wherein the step of extracting geometric parameters of occluded vessels and of the composition parameters of the occlusion from the clinical images, provides for processing the clinical images using a grayscale analysis algorithm to extract the composition parameters of the occlusion.

10. Method according to any one of the preceding claims, wherein the step of calculating the likelihood of success (5) of the thrombectomy surgery with removal of the occlusion by processing the indicator parameters using the predictive algorithm (P) comprises the sub-step of:
- calculating the likelihood of an occlusal fracture occurring by processing the indicator parameters using the predictive algorithm (P).

11. Method according to any one of the preceding claims, wherein:
- the step of training the predictive algorithm (P) by processing the data using machine learning techniques on a predefined number of finite element numerical simulations of the thrombectomy procedure provides that the thrombectomy surgery employs at least one thrombectomy procedure and at least one respective biomedical device;
- prior to the step of calculating the likelihood of success (5) of thrombectomy surgery, there is a further step of:
- selecting (6) at least one thrombectomy procedure and at least one respective biomedical device to be used in the thrombectomy surgery.

## Patentansprüche

1. Verfahren zum Schätzen der Erfolgswahrscheinlichkeit eines Thrombektomie-Eingriffs in Echtzeit, umfassend die folgenden Schritte:
- Erstellen einer aus Thrombektomiemustern erzeugten Datenbank und Trainieren eines prädiktiven Algorithmus (P) mit diesen Daten unter Verwendung einer der Datenbank zugeordneten Verarbeitungseinheit;
- Erfassen von klinischen Bildern (1), die sich auf die okkludierten Gefäße eines Patienten beziehen, und Extrahieren der geometrischen Parameter der okkludierten Gefäße (2) und der Zusammensetzungsparameter der Okklusion (3) aus den besagten klinischen Bildern;
- Erzeugen eines dreidimensionalen Modells (4) der okkludierten Gefäße und der Okklusion durch Verarbeiten der geometrischen Parameter der okkludierten Gefäße (2) und der Zusammensetzungsparameter der Okklusion (3) unter Verwendung der Verarbeitungseinheit;
- Auswählen von Indikatorparametern für den Thrombektomie-Eingriff durch Verarbeiten des dreidimensionalen Modells der okkludierten Gefäße und der Okklusion;
- Berechnen der Erfolgswahrscheinlichkeit (5) des Thrombektomie-Eingriffs mit Entfernung der Okklusion durch Verarbeiten der Indikatorparameter unter Verwendung des prädiktiven Algorithmus (P), **dadurch gekennzeichnet, dass** der Schritt des Erstellens einer unter Verwendung von Thrombektomiemustern erzeugten Datenbank und des Trainierens eines prädiktiven Algorithmus (P) mit den besagten Daten unter Verwendung einer der Datenbank zugeordneten Verarbeitungseinheit das Trainieren des prädiktiven Algorithmus (P) durch Verarbeiten der Daten unter Verwendung von Techniken des maschinellen Lernens an einer vordefinierten Anzahl von numerischen Finite-Elemente-Simulationen des Thrombektomie-Eingriffs umfasst.

2. Verfahren nach Anspruch 1, wobei der Schritt des Auswählens von Indikatorparametern die Teilschritte umfasst:
- Berechnen von morphologischen Parametern der Gefäße durch Analyse der Mittellinie der okkludierten Gefäße und ihrer Durchmesser;
- Berechnen von morphologischen Parametern der Okklusion durch Analyse der Länge, des Durchmessers und der Zusammensetzung der Okklusion.

3. Verfahren nach Anspruch 2, wobei die morphologischen Parameter der Gefäße einen oder mehrere Parameter umfassen, die ausgewählt sind aus den Winkeln, die sich dort bilden, wo die Arteria carotis interna (ACI) in die Arteria cerebri anterior (ACA) und die Arteria cerebri media (ACM) bifurziert, den mittleren Durchmessern der Arteria cerebri media (ACM) und der Arteria cerebri anterior (ACA) und charakteristischen Parametern des Karotissiphons der Arteria carotis interna (ACI).

4. Verfahren nach Anspruch 3, wobei die charakteristischen Parameter des Karotissiphons der Arteria carotis interna (ACI) einen oder mehrere Parameter umfassen, die ausgewählt sind aus den Krümmungsradien der Schleifen, der Länge der Schleifen, der Tortuosität der Schleifen, den Winkeln zwischen den Schleifen und den mittleren Durchmessern der Schleifen.

5. Verfahren nach Anspruch 1, wobei der Schritt des Auswählens von Indikatorparametern die Teilschritte umfasst:
- Berechnen von charakteristischen Parametern der Geometrie der Gefäße durch Analysieren der gesamten Geometrie der okkludierten Gefäße unter Verwendung der Level-Set-Technik;
- Berechnen von morphologischen Parametern der Okklusion durch Analyse von Länge, Durchmesser und Zusammensetzung der Okklusion.

6. Verfahren nach Anspruch 5, wobei der Schritt des Berechnens von charakteristischen Parametern der Gefäßgeometrie die Teilschritte umfasst:
- Definieren eines festen Volumens, das so skaliert ist, dass es alle möglichen vaskulären Geometrien von Patienten aufnehmen kann;
- Discretisieren des Volumens in einem Gitter gemäß einer Sensitivitätsanalyse;
- Platzieren jeder rekonstruierten Gefäßgeometrie in dem Gitter in dem dreidimensionalen Modell der okkludierten Gefäße und der Okklusion und Berechnen einer Matrix mit der gleichen Größe wie die Anzahl der Gitterpunkte durch Messen der Abstände zwischen jedem Gitterpunkt und dem nächstgelegenen Punkt der Gefäßgeometrie;
- Berechnen der charakteristischen Parameter der Geometrie der Gefäße durch Verarbeiten der Matrix unter Verwendung einer Technik der Hauptkomponentenanalyse.

7. Verfahren nach einem der vorhergehenden Ansprüche, wobei die klinischen Bilder (1) Bilder umfassen, die durch Computertomographie und/oder Magnetresonanztomographie gewonnen wurden.

8. Verfahren nach einem der vorhergehenden Ansprüche, wobei:
- die geometrischen Parameter der okkludierten Gefäße Mittellinie und Durchmesser für jedes okkludierte Gefäß umfassen;
- die Zusammensetzungsparameter der Okklusion (3) Position, Länge und Zusammensetzung der Okklusion umfassen.

9. Verfahren nach Anspruch 8, wobei der Schritt des Extrahierens von geometrischen Parametern von okkludierten Gefäßen und der Zusammensetzungsparameter der Okklusion aus den klinischen Bildern das Verarbeiten der klinischen Bilder unter Verwendung eines Graustufenanalysealgorithmus vorsieht, um die Zusammensetzungsparameter der Okklusion zu extrahieren.

10. Verfahren nach einem der vorhergehenden Ansprüche, wobei der Schritt des Berechnens der Erfolgswahrscheinlichkeit (5) des Thrombektomie-Eingriffs mit Entfernung der Okklusion durch Verarbeiten der Indikatorparameter unter Verwendung des prädiktiven Algorithmus (P) den Teilschritt umfasst:
- Berechnen der Wahrscheinlichkeit des Auftretens einer Okklusionsfraktur durch Verarbeiten der Indikatorparameter unter Verwendung des prädiktiven Algorithmus (P).

11. Verfahren nach einem der vorhergehenden Ansprüche, wobei:
- der Schritt des Trainierens des prädiktiven Algorithmus (P) durch Verarbeiten der Daten unter Verwendung von Techniken des maschinellen Lernens an einer vordefinierten Anzahl von numerischen Finite-Elemente-Simulationen des Thrombektomieverfahrens vorsieht, dass der Thrombektomie-Eingriff mindestens ein Thrombektomieverfahren und mindestens eine jeweilige biomedizinische Vorrichtung einsetzt;
- vor dem Schritt des Berechnens der Erfolgswahrscheinlichkeit (5) des Thrombektomie-Eingriffs ein weiterer Schritt vorhanden ist:
- Auswählen (6) von mindestens einem Thrombektomieverfahren und mindestens einer jeweiligen biomedizinischen Vorrichtung, die bei dem Thrombektomie-Eingriff zu verwenden sind.

## Revendications

1. Procédé pour estimer en temps réel la probabilité de succès d'une intervention de thrombectomie, comprenant les étapes suivantes :
- création d'une base de données générée à partir de modèles de thrombectomie et apprentissage d'un algorithme prédictif (P) avec ces données à l'aide d'une unité de traitement associée à la base de données ;
- acquisition d'images cliniques (1) relatives aux vaisseaux occlus d'un patient et extraction, à partir desdites images cliniques, des paramètres géométriques des vaisseaux occlus (2) et des paramètres de composition de l'occlusion (3) ;
- génération d'un modèle tridimensionnel (4) des vaisseaux occlus et de l'occlusion par traitement des paramètres géométriques des vaisseaux occlus (2) et des paramètres de composition de l'occlusion (3) à l'aide de l'unité de traitement ;
- sélection de paramètres indicateurs pour l'intervention de thrombectomie par traitement du modèle tridimensionnel des vaisseaux occlus et de l'occlusion ;
- calcul de la probabilité de succès (5) de l'intervention de thrombectomie avec retrait de l'occlusion par traitement des paramètres indicateurs à l'aide de l'algorithme prédictif (P), **caractérisé en ce que** l'étape de création d'une base de données générée à l'aide de modèles de thrombectomie et d'apprentissage d'un algorithme prédictif (P) avec lesdites données à l'aide d'une unité de traitement associée à la base de données, comprend l'apprentissage de l'algorithme prédictif (P) par traitement des données à l'aide de techniques d'apprentissage automatique sur un nombre prédéfini de simulations numériques par éléments finis de l'intervention de thrombectomie.

2. Procédé selon la revendication 1, dans lequel l'étape de sélection de paramètres indicateurs comprend les sous-étapes de :
- calcul de paramètres morphologiques des vaisseaux par analyse de la ligne médiane des vaisseaux occlus et de leurs diamètres ;
- calcul de paramètres morphologiques de l'occlusion par analyse de la longueur, du diamètre et de la composition de l'occlusion.

3. Procédé selon la revendication 2, dans lequel les paramètres morphologiques des vaisseaux comprennent un ou plusieurs paramètres choisis parmi les angles qui se forment là où l'Artère Carotide Interne (ACI) bifurque sur l'Artère Cérébrale Antérieure (ACA) et l'Artère Cérébrale Moyenne (ACM), les diamètres moyens de l'Artère Cérébrale Moyenne (ACM) et de l'Artère Cérébrale Antérieure (ACA), et des paramètres caractéristiques du siphon carotidien de l'Artère Carotide Interne (ACI).

4. Procédé selon la revendication 3, dans lequel les paramètres caractéristiques du siphon carotidien de l'Artère Carotide Interne (ACI) comprennent un ou plusieurs paramètres sélectionnés parmi les rayons de courbure des boucles, la longueur des boucles, la tortuosité des boucles, les angles entre les boucles et les diamètres moyens des boucles.

5. Procédé selon la revendication 1, dans lequel l'étape de sélection de paramètres indicateurs comprend les sous-étapes de :
- calcul de paramètres caractéristiques de la géométrie des vaisseaux par analyse de la géométrie entière des vaisseaux occlus à l'aide de la technique des ensembles de niveaux ;
- calcul de paramètres morphologiques de l'occlusion, par analyse de la longueur, du diamètre et de la composition de l'occlusion.

6. Procédé selon la revendication 5, dans lequel l'étape de calcul de paramètres caractéristiques de la géométrie du vaisseau comprend les sous-étapes de :
- définition d'un volume fixe mis à l'échelle pour pouvoir s'adapter à toutes les géométries vasculaires possibles du patient ;
- discrétisation du volume en une grille selon une analyse de sensibilité ;
- placement de chaque géométrie vasculaire reconstruite dans la grille dans le modèle tridimensionnel des vaisseaux occlus et de l'occlusion et calcul d'une matrice de la même taille que le nombre de points de grille en mesurant les distances entre chaque point de grille et le point le plus proche de la géométrie vasculaire ;
- calcul des paramètres caractéristiques de la géométrie des vaisseaux par traitement de la matrice à l'aide d'une technique d'analyse en composantes principales.

7. Procédé selon l'une quelconque des revendications précédentes, dans lequel les images cliniques (1) comprennent des images obtenues par tomodensitométrie et/ou imagerie par résonance magnétique.

8. Procédé selon l'une quelconque des revendications précédentes, dans lequel :
- les paramètres géométriques des vaisseaux occlus comprennent la ligne médiane et le diamètre pour chaque vaisseau occlus ;
- les paramètres de composition de l'occlusion (3) comprennent la position, la longueur et la composition de l'occlusion.

9. Procédé selon la revendication 8, dans lequel l'étape d'extraction des paramètres géométriques des vaisseaux occlus et des paramètres de composition de l'occlusion à partir des images cliniques, prévoit le traitement des images cliniques à l'aide d'un algorithme d'analyse en niveaux de gris pour extraire les paramètres de composition de l'occlusion.

10. Procédé selon l'une quelconque des revendications précédentes, dans lequel l'étape de calcul de la probabilité de succès (5) de l'intervention de thrombectomie avec retrait de l'occlusion par traitement des paramètres indicateurs à l'aide de l'algorithme prédictif (P) comprend la sous-étape de :
- calcul de la probabilité de survenue d'une fracture de l'occlusion par traitement des paramètres indicateurs à l'aide de l'algorithme prédictif (P).

11. Procédé selon l'une quelconque des revendications précédentes, dans lequel :
- l'étape d'apprentissage de l'algorithme prédictif (P) par traitement des données à l'aide de techniques d'apprentissage automatique sur un nombre prédéfini de simulations numériques par éléments finis de la procédure de thrombectomie prévoit que l'intervention de thrombectomie emploie au moins une procédure de thrombectomie et au moins un dispositif biomédical respectif ;
- préalablement à l'étape de calcul de la probabilité de succès (5) de l'intervention de thrombectomie, il y a une étape supplémentaire de :
- sélection (6) d'au moins une procédure de thrombectomie et d'au moins un dispositif biomédical respectif à utiliser dans l'intervention de thrombectomie.
